# EUROPEAN PATENT SPECIFICATION

(11) **EP 0 610 356 B1**
(45) Date of publication and mention of the grant of the patent: **31.01.2001**
(21) Application number: 92922817.9
(22) Date of filing: 19.10.1992
(51) Int. Cl.: A61K 47/48

(54) **TREATMENT OF BACTERIAL DYSENTERY**
BEHANDLUNG BAKTERIELLER DYSENTERIE
TRAITEMENT DE LA DYSENTERIE BACTERIENNE

(30) Priority: 18.10.1991 US 778732
(43) Date of publication of application: 17.08.1994
(60) Divisional application: 00101263.2
(73) Proprietor: SYNSORB Biotech Inc., Calgary, Alberta T2N 3P5 (CA)
(72) Inventor: ARMSTRONG, Glen, D., Edmonton, Alberta T6C 1P9 (CA); RATCLIFFE, R., Murray, Carlsbad, CA 92008 (US)
(74) Representative: Nash, David Allan
(86) International application number: PCT/US92/08929
(87) International publication number: WO 93/08209

(56) References cited:
- WO-A-86/04064
- GB-A- 2 223 579
- US-A- 4 863 852
- FEBS LETTERS, vol.258, no.2, 4 December 1989 pages 320 - 322 RYD M. ET AL.
- CHEMISTRY AND PHYSICS OF LIPIDS, vol.42, 1986 pages 153 - 172 KARLSSON K.
- THE JOURNAL OF INFECTIOUS DISEASES, vol.164, no.6, December 1991 pages 1160 - 1167 ARMSTRONG G.D. ET AL.
- Methods in Enzymology, Vol. 34, issued 1974, H. PARIKH et al., "Ganglioside-Agarose and Cholera Toxin", pp. 610-619.
- Proceedings of the National Academy of Sciences (USA), Vol. 87, issued October 1990, T. WADDELL et al., "Induction of Verotoxin Sensitivity in Receptor-Deficient Cell Lines Using the Receptor Glycolipid Globotriosylceramide", pp. 7898-7901, see entire document.
- The Journal of Biological Chemistry, Vol. 262, No. 18, issued 25 June 1987, C.A. LINGWOOD et al., "Glycolipid Binding of Purified and Recombinant Escherichia coli Produced Verotoxin in Vitro", pp. 8834-8839, see entire document.
- Nephron, Vol. 51, issued 1989, B. BOYD et al., "Verotoxin Receptor Glycolipid in Human Renal Tissue", pp. 207-210, see entire document.
- Infection and Immunity, Vol. 58, No. 8, issued August 1990, T. DAIKOKU et al., "Partial Purification and Characterization of the Enterotoxin Produced by Campylobacter jejuni", pp. 2414-2419, see entire document.
- Journal of Experimental Medicine, Vol. 163, issued June 1986, M. JACEWICZ et al., "Pathogenesis of Shigella Diarrhea", pp. 1391-1404, see entire document.
- Biochemical and Biophysical Research Communications, Vol. 152, No. 2, issued 29 April 1988, T. WADDELL et al., "Globotriosyl Ceramide is Specifically Recognized by the Escherichia coli Verocytotoxin", pp. 674-679, see entire document.
- The Journal of Biological Chemistry, Vol. 264, No. 21, issued 25 July 1989, S. DEGRANDIS et al., "Globotetraosylceramide is Recognized by the Pig Edema Disease Toxin", pp. 12520-12525, see entire document.
- Infection and Immunity, Vol. 58, No. 3, issued March 1990, J.E. SAMUEL et al., "Comparison of the Glycolipid Receptor Specificities of Shiga-Like Toxin Type II and Shiga-Like Toxin Type II Variants", pp. 611-618, entire document.
- Journal of Bacteriology, Vol. 170, No. 9, issued September 1988, D.L. WEINSTEIN et al., "Cloning and Sequencing of a Shiga-Like Toxin Type II Variant from an Escherichia coli Strain Responsible for Edema Disease of Swine", pp. 4223-4230, see entire document.
- Advances in Experimental Medicine and Biology, Vol. 125, issued 1980, J.L. TAYOT et al., "Isolation of Cholera Toxin by Affinity Chromatography on Porous Silica Beads with Covalently Coupled Ganglioside GM1", pp. 471-478, see entire document.
- Journal of General Microbiology, Vol. 136, Part 9, issued 1990, R.S. DUBEY et al., "Purification of E1 Tor Cholera Enterotoxins and Comparisons with Classical Toxin", pp. 1839-1847, see entire document.
- Journal of Experimental Medicine, Vol. 165, issued March 1987, U. GALILI et al., "The Human Natural Anti-Gal IgG", pp. 693-704, see entire document.
- Armstrong et al, J.Infect.Dis., vol. 164, 1994, p. 1160 - 1166

## Description

### Technical Field

The invention relates to compositions for treatment of diarrhea caused by bacterial infection.

### Background Art

Diarrhea caused by strains of pathogenic *E. coli* has been found to be associated with the production of a variety of enterotoxins. Some pathogenic *E. coli* enterohemorrhagic produce enterotoxins that are closely related to the shiga toxin associated with Shigella-caused dysentery. The first member of the family of shiga-like toxins (SLT) to be isolated was cytotoxic for African Green Monkey (Vero) cells and was originally called verotoxin. Since its structural similarity to shiga toxin has been established by sequencing of the relevant genes, this toxin is now more commonly called shiga-like toxin I (SLTI). See, for example, Calderwood, S.B., et al., Proc Natl Acad Sci USA (1987) 84:4364-4368; Jackson, M.P., et al., Microb Pathog (1987) 2:147-153; Strockbine, N.A., et al., J Bacteriol (1988) 170:1116-1122.

Additional members of the SLT family have subsequently been isolated that can be distinguished serologically, on the basis of gene sequence or host specificity (Gannon, V.P.J., et al., J Gen Microbiol (1990) 136:1125-1135; Weinstein, D.L., et al., J Bacteriol (1988) 170:4223-4230; Ito, H., et al., Microb Pathog (1990) 8:47-60; Head, S.C., et al., FEMS Microbiol Lett (1988) 51:211-216; Schmitt, C.K., et al., Infect Immun (1991) 59:1065-1073; Scotland, S.M., et al. Lancet (1985) ii:885-886; Oku, Y., et al., Microb Pathog (1989) 6:113-122. Various types of SLTII have been described and have been assigned various designations depending on the strain of *E. coli* from which they are isolated and the hosts they inhabit. Thus, variants have been designated SLTII; vtx2ha; SLTIIvh; vtx2hb; SLTIIc; SLTIIvp and so forth.

All of the SLT's are multimeric proteins composed of an enzymatic (A) subunit and multiple (B) subunits responsible for toxin binding to receptors on host tissues. The binding B oligomers of SLTI, SLTII and SLTIIvh recognize host cell globoseries glycolipid receptors containing at minimum the disaccharide subunit aGal(1-4)βGal at the non-reducing terminus; SLTIIvp has been shown to bind to the receptors containing this subunit but not necessarily at the non-reducing end (Samuel, J.E., et al., Infect Immun (1990) 58:611-618; Boyd, B., et al., Nephron (1989) 51:207-210; DeGrandis, S., et al., J Biol Chem (1989) 264:12520-12525; Waddell, T., et al., Biochem Biophys Res Comm (1988) 152:674-679; Lingwood, C.A., et al., J Biol Chem (1987) 262:8834-8839; Waddell, T., et al., Proc Natl Acad Sci USA (1990) 87:7898-7901; Cohen, A., et al., J Biol Chem (1987) 262:17088-17091; Jacewicz, M., et al., J Exp Med (1986) 163:1391-1404; Lindberg, A.A., et al., J Biol Chem (1987) 262:1779-1785.

SLT activity has been detected in stool samples of symptomatic patients (Karmali, M.A., et al., J Clin Microbiol (1988) 22:614-619; Maniar, A.C., et al., J Clin Microbiol (1990) 28:134-135). However, there is difficulty in detecting the presence of SLTs clinically since these are very potent toxins present in low concentrations. In order to assure detection, the SLT present in the sample must be concentrated to enhance the reliability of the assay. Present diagnostic procedures are technically demanding, time consuming and of limited practical use in the clinical setting. Thus there is a clear need for improved diagnostic clinically practical and rapid procedures.

Also, antibiotics are not recommended for treatment of enterohemorrhagic *E. coli* infection (Robson, W.L.M., et al., J Pediatr (1990) 117:675-676) and the use of antimotility drugs also appears to be counterproductive (Cimolai, N., et al., J Pediatr (1990) 117:676. There is, therefore, also a clear need for new and effective therapeutic agents.

WO-A-86/04064 discloses a compound and a composition for therapeutic or diagnostic use and the use of such a compound for the therapeutic treatment and isolation of the toxin from *Shigella dysenteriae.*

The *E. coli* organism and Shiga toxin bind Galα(1-4)Gal in a thin-layer chromatogram overlay assay. See "The Chemistry and Physics of Lipids vol 42, 1986, p152-172".

Weinstein *et al.* (J. Bacteriol. vol 170, 1988, p. 4223-4230) describes the cloning and sequencing of a Shiga-like toxin type II variant from an *E. coli* strain responsible for Edema disease of swine.

It has now been found that artificial substrates containing the αGal(1-4)βGal (Pₗ disaccharide) subunit and more preferably the αGal(l-4)βGal(1-4)βGIcNAc (Pₗ trisaccharide) or aGal(1-4)BGal(1-4)βGlc (Pₖ trisaccharide) subunit are effective in detecting and neutralizing members of the SLT family under conditions necessary to effect recovery of the patient and as such represent novel therapeutic tools in the treatment of *E. coli*-mediated dysentery.

### Disclosure of the Invention

The invention provides compositions useful in the therapy of *E. coli*-caused enterotoxic bacterial and related infections that present clinically as severe diarrhea, hemorrhagic colitis, hemolytic uremic syndrome or thrombotic thrombocytopenic purpura. According to a first aspect of the present invention, there is provided a pharmaceutical composition useful in treating enteric infections mediated by SLT which composition comprises as active ingredient a pharmaceutically acceptable solid inert affinity support capable of being elimiated from the gastrointestinal tract which support has an affinity ligand covalently attached thereto through a spacer arm wherein said ligand is characterized as an oligosaccharide containing, at the non-reducing terminus thereof, the αGal(1-4)βGal(1-4)βGlcNAc trisaccharide or the αGal(1-4)βGal(1-4)βGlc trisaccharide which binds the SLT, in admixture with a pharmaceutically acceptable excipient.

Preferably, the oligosaccharide is the αGal(1-4)βGal(1-4)βGlcNAc trisaccharide or the αGal(1-4)βGal(1-4)βGlc trisaccharide.

According to a second aspect of the present invention, there is provided an agent for use in a method of treating a subject to prevent or ameliorate an enteric infection mediated by SLT, wherein said agent is a pharmaceutically acceptable solid inert affinity support capable of being eliminated from the gastrointestinal tract which support has an affinity ligand covalently attached thereto through a spacer arm wherein said ligand is characterized as an oligosaccharide containing, at the non-reducing terminus thereof, the αGal(1-4)βGal(1-4)βGlcNAc trisaccharide or the αGal(1-4)βGal(1-4)βGlc trisaccharide which binds the SLT.

Figures 1 A and B demonstrate the toxicity of bacterial extracts obtained using polymixin-B with respect to their ability to kill Vero cells in the presence and absence of various SYNSORBS.

Figures 2 A and B demonstrate the toxicity of bacterial extracts obtained using lysozyme with respect to their ability to kill Vero cells in the presence and absence of various SYNSORBS.

Figure 3 A demonstrates that as little as 10 mg of Pₖ trisaccharide SYNSORB removes >90% of SLT toxins from bacterial extracts.

B demonstrates that the binding of the SLT toxins occurred within 5 minutes of mixing extracts with the Pₖ SYNSORB.

Figure 4 demonstrates the difficulty in eluting the bound I¹²⁵ labelled SLTI from various SYNSORBs utilizing a variety of eluants.

Figure 5 demonstrates that >90% SLTI, SLTII/IIc and SLTII activity was neutralized by co-incubation of Vero cells and SLT extracts for three days, with as little as 10 mg of Pₖ trisaccharide SYNSORB.
The subunit is bound through a spacer arm preferably through a linking arm such as that described by Lemieux, R.U., et al., J Am Chem Soc. (1975) 97:4076-4083; to a solid, inert support that can be easily eliminated from the gastrointestinal system. An inert silica matrix embodiments of which are commercially available as "SYNSORB™" are preferred.

### Modes of Carrying Out the Invention

The trisaccharides may be provided as a portion of a larger oligosaccharide coupled to a solid support or coupled directly, preferably through a linking arm such as that described by Lemieux, R.U., et al., J Am Chem Soc (1975) 97:4076-4083.

As used herein, "shiga-like toxin" or "SLT" refers to group of toxins produced by enterohemorrhagic *E. coli* that resemble the Shigella-produced shiga toxins as is commonly understood in the art. These toxins comprise an enzymatically active A subunit and a multimeric receptor binding B subunit. Such SLTs include SLTI and the various grouped toxins designated in the art SLTII.

Rapid, tight binding of SLT's to Pₗ trisaccharide or Pₖ trisaccharide is demonstrated by the verocytoxicity neutralization and I¹²⁵ binding assays contained herein.

The SYNSORB™s employed were obtained from Chembiomed (Edmonton, Canada). In each case the 8-methoxycarbonyloctyl glycoside of the respective hapten is activated and ligated to a silylaminated solid support, wherein the matrix is comprised of SiO₂, followed by acetylation of the remaining amine groups on the solid support. These formulations are sold commercially as "SYNSORB™"s:
"Pₗ-di," which contains 0.60 µmole/g αGal(1-4)βGal disaccharide;
"Pₗ-tri," which contains 0.91 µmole/g αGal(1-4)βGal(1-4)βGlcNAc trisaccharide;
"Pₖ-tri," which contains 0.74 µmole/g αGal(1-4)βGal(1-4)βGlc trisaccharide;
"Linear B like tri," which contains 0.47 µmole/g αGal(1-3)βGal(1-4)βGlcNAc trisaccharide;
"Linear B like di," which contains 0.66 µmole/g αGal(1-3)βGal disaccharide;
"Glucose mono," which contains 1.0 µmol B-glucose; 8-methoxycarbonyoctanol activated and ligated to the silylated solid support.
"ASA" which contains only the acetylated silylaminated (ASA) hydrophobic 8-methoxycarbonyloctyl linkage arm.

In coupling the relevant subunits to supports, it is preferable that a spacer arm be used so that the affinity ligand is permitted ready access to the toxin to be coupled. A particularly preferred spacer arm is the 8-methoxycarbonyloctyl-activated (MCO) form of the oligosaccharide to be coupled. This spacing appears to be particularly advantageous, and thus alternative chemistries which provide the same spacing may also used. Any spacer arm which is of a corresponding length and is provided functional groups at each terminus for reaction with the disaccharide, trisaccharide or oligosaccharide on the one hand and the solid support on the other may be used; such spacer arms "correspond" to MCO. The nature of these functional groups is understood by those of ordinary skill in the art to depend on the nature of the oligosaccharide and the nature of the solid support used.

Compositions of the invention for use in therapeutic applications may be supplied wherein the trisaccharide subunit or an oligomer saccharide containing it is coupled to a nontoxic carrier, such as a liposome, biocompatible polymer, or carrier analogous to the above-referenced SYNSORB™s.

The active ingredient may be formulated in standard pharmaceutical compositions for administration to the patient. Typically, the patient will be afflicted with a diarrhetic condition, such as hemorrhagic colitis due to enterohemorrhagic *E. coli* infection, and the target SLT will be present in the intestinal tract. Thus, a suitable mode of administration is through oral administration or other means of direct application to the gastrointestinal tract. The correct dosage range will depend on the severity of the infection, the mode of administration, the mode of formulation, and the judgment of the attending practitioner.

As shown in Example 3, pharmaceutical formulations are available that survive the conditions of the digestive tract. The oligosaccharides responsible for toxin binding and coupled to the SYNSORBs through a spacer arm are resistant to the potentially degradative conditions found in the digestive tract. Thus, the ability of the derivatized affinity ligands is unimpaired by incubation under acidic conditions approximating those found in the stomach and in the presence of the degradative enzymes and higher pH conditions found in the small intestine. It is required for successful pharmaceutical compositions that they pass through the stomach and the intestines of prior to encountering the sites of infection.

The SYNSORB compositions themselves can be used as pharmaceutical compositions since the solid support is physiologically innocuous. In the commercially available SYNSORB, the solid support is silica, a commonly used drying agent in such foods as dairy creamers. Thus, the the compositions tested, per se, are useful pharmaceutical compositions.

The effect of the compositions of the invention in neutralizing SLTs can be measured by comparing activity of SLT with and without treatment with the compositions. Activity of the SLTs can be assayed by taking advantage of the toxicity of these compounds to Vero cells. Vero cells (ATCC CCL81) can be obtained from the American-Type Culture Collection, Rockville, MD. These are maintained at 37°C/5% CO₂ in minimal essential medium with Earl's salts (MEM, Gibco BRL, Gaithersburg, MD) containing 3% fetal bovine serum (FBS). Confluent Vero cell monolayers are disrupted using 0.25% tissue-culture grade trypsin and approximately 10⁵ cells in 200 µl FBS-supplemented MEM are added to each well of a 96-well microtiter plate. The plates are incubated overnight at 37°C/5% CO₂.

The samples to be tested, and suitable controls are added to the various wells and the plates are incubated for 2-3 days at 37°C/5% CO₂. Cytotoxic effects are readily visible in successful candidate wells as compared to control wells. The results can be quantitated by aspirating the remaining liquid from each of the wells and fixing the Vero cells which remain viable with 95% methanol and staining with Geimsa stain. The results are recorded using a microtiter plate reader set at a wavelength of 620 nm, as described by Samuel, J.E., Infect Immun (1990) 58:611-618 (supra). The absorbance data are plotted versus the logarithm of the dilution of the candidate test solution. The dilution of samples resulting in 50% destruction (CD₅₀) of the monolayers is determined by extrapolation from the resulting Vero cell killing curves.

The following examples are intended to illustrate but not to limit the invention.

### Example 1

### SYNSORB - Verocytotoxicitv Neutralization Assays

*E. coli* strains 0157:H-(E32511), which produces SLTII/SLTIIc and 026:H11(H19) which produces SLTI only, or strain C600(933W), which produces SLTII only, were grown overnight at 37°C on tryptic soy broth (Difco, Detroit, MI) agar plates. Polymyxin and lysozyme extracts were prepared as described previously [Karmali, M.A., et al., J Clin Microbiol (1985) 22:614-619 and Head, S., et al., Infect Immunol (1990) 58:1532-1537)].

The first neutralization assay was designed to test the ability of SYNSORBs to absorb SLT activity from the *E. coli* extracts. The assay involved incubating 1 mL of the *E. coli* extracts for 30 min. at room temperature in 1.5 mL microcentrifuge tubes (Fisher) with 2 to 50 mg SYNSORB on an end-over-end rotator. The tubes were then removed from the apparatus and after the SYNSORB had settled to the bottom (a few seconds), serial five-fold dilutions of the absorbed extracts were prepared in unsupplemented MEM. Twenty (20) µL of each dilution was added to the appropriate wells in 96 well microtiter plates containing Vero cells. Bacterial extracts to which no SYNSORB was added served as controls. Once cytotoxic effects became apparent (2 to 3 days in the incubator) the growth medium was aspirated from each of the wells and Vero cells which remained viable were fixed with 95% methanol and stained with Giemsa stain (Fisher). The results were then recorded using a microtiter plate reader set at a wavelength of 620 nm as described previously [Samuel et al., Infect Immun. 58:611-618 (1990)]. The absorbance data were then plotted versus the logarithm of the extract dilution. The dilution of the extracts resulting in 50% destruction (CD₅₀) of the monolayers was determined by extrapolation from the resulting Vero cell killing curves. Individual experiments were always performed in duplicate and, unless otherwise indicated, repeated at least two times. The percentage of neutralization was computed from the equation: 100-(100[CD₅₀ oligosaccharide SYNSORB-treated extracted + CD₅₀ acetylated silyl-aminated (ASA) SYNSORB-treated extract]). The non-parametric Mann-Whitney test using the two-tailed statistic T was employed to compute the significance level of difference between groups of independent observations [Altman, D.G., Practical statistics for medical research, 1st ed. New York, Chapman and Hall: 179-228 (1991)].

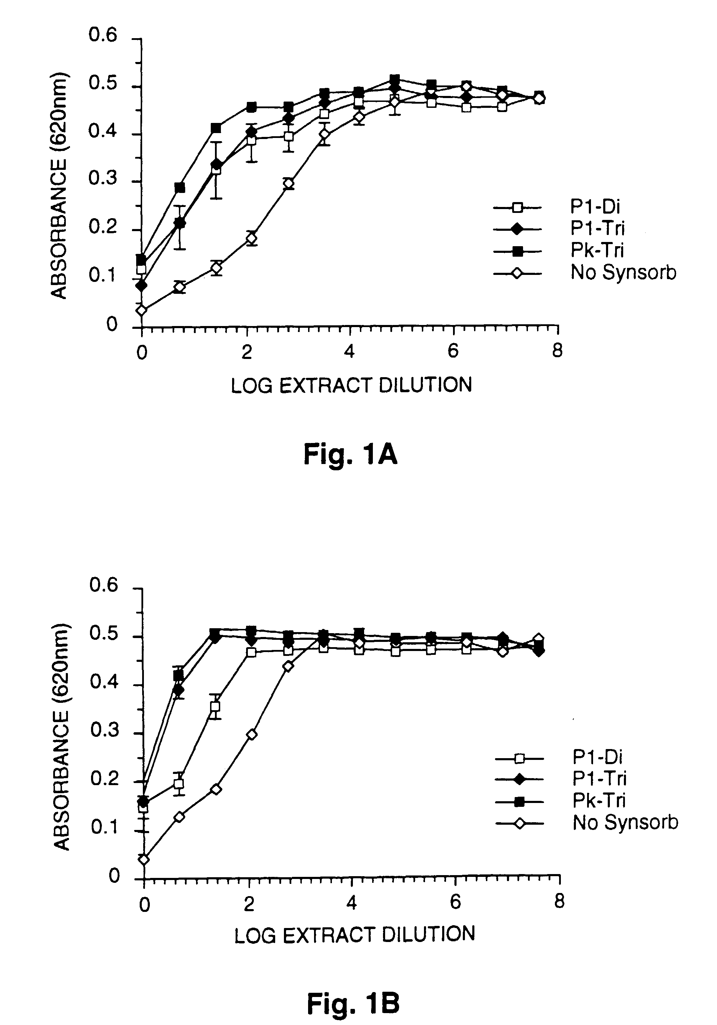

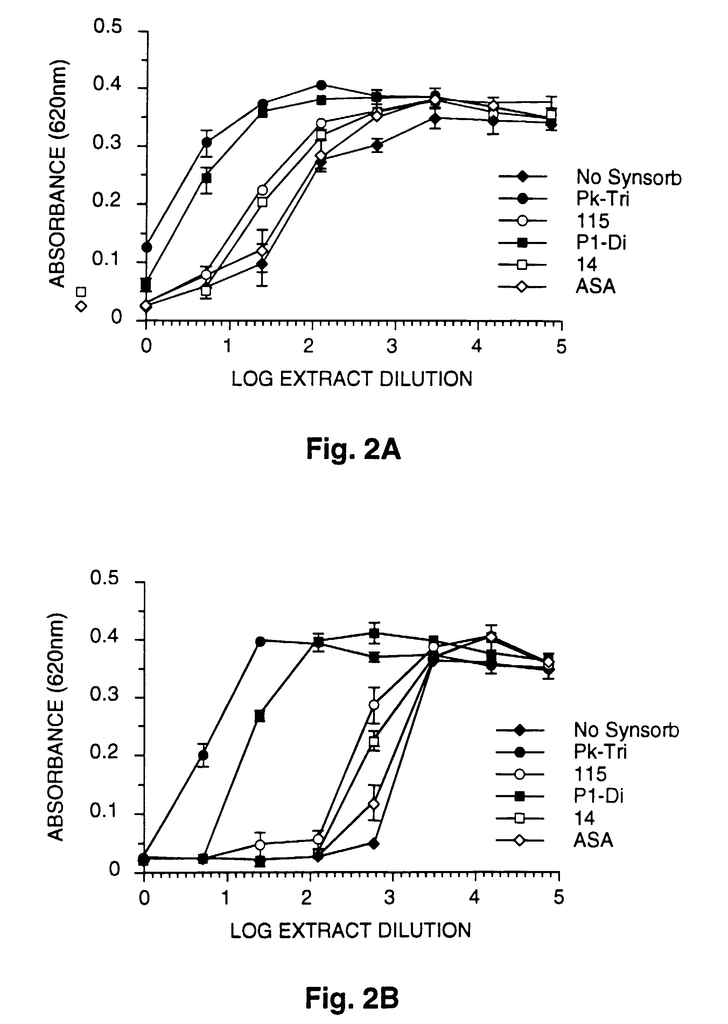

The second neutralization assay (co-incubation assay) was designed to test the ability of Pk trisaccharide SYNSORB to protect Vero cells from SLT activity over 3 days at 37°C. This assay involved incubating 180 µL of serial five-fold dilutions of polymyxin extracts in ethylene oxide-sterilized 1.5 mL microcentrifuge tubes each containing 2, 5 or 10 mg of Pk trisaccharide SYNSORB. After 1 h incubation with SYNSORB, the entire contents of each microcentrifuge tube were added to Vero cells monolayers in microtiter plates prepared as described above. The microtiter plates prepared as described above. The microtiter plates were then incubated at 37°C for 3 days and the results of the experiment were recorded as described above (Figures 1 and 2).

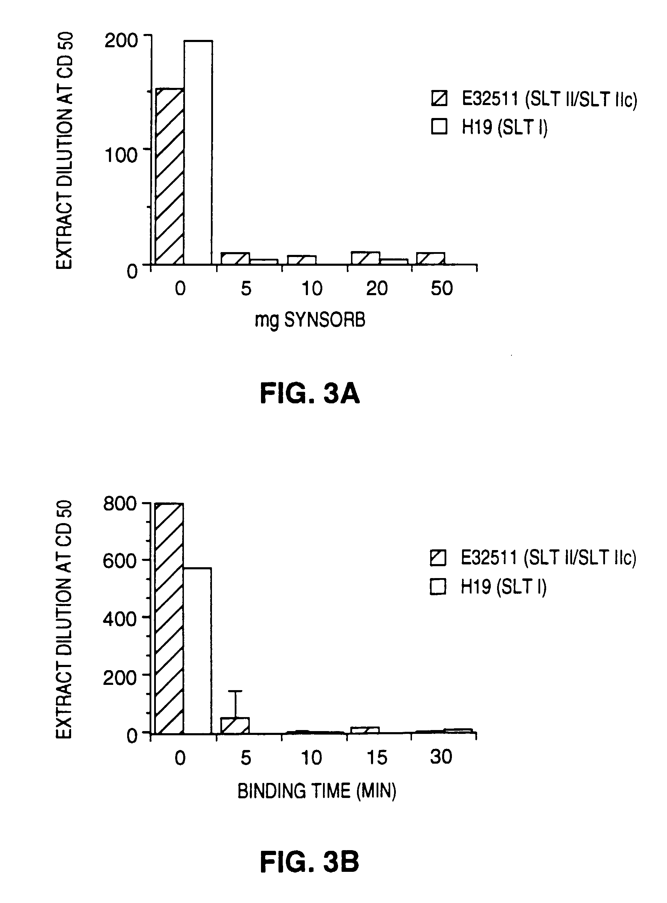

The foregoing determination was repeated using varying amounts of Pk-tri and various times of incubation, with the results shown in Figures 3A and 3B. As shown in Figure 3A, as little as 5 mg SYNSORB was capable of neutralizing the activity of the extracts of both E32511 and H19 strains; similarly, as shown in Figure 3B, only about 5 min incubation was required to achieve this result in either extract.

### Example 2

### Iodinated SLTI Binding Assay

Purified SLTI was iodinated in 12 x 75 mm acid-washed glass culture tubes coated with 40 µg of Iodo Gen (Pierce Chemical Co., Rockford, IL). About 6 µg of purified SLTI was incubated for 1 min with 20 MBq ¹²⁵-I labeled sodium iodide in 100 µl PBS. The reaction mixture was passed through a glass wool-plugged Pasteur pipette into 200 µl PBS containing a solution of cysteine (1 mg/ml) in PBS as described by Armstrong, G.D., et al., Infect Immunol (1987) 55:1294-1299. After 1 min, 200 µl of PBS containing 1% BSA was added to the mixture and the iodinated SLTI was purified by passing the solution through a 1 cm x 30 cm Sephadex-G 25 gel filtration column with 0.1% BSA in PBS. The efficiency of the iodination reaction was determined by measuring the number of counts that were incorporated into trichloroacetic acid precipitated protein. Aliquots of the iodinated SLTI were stored at -90°C.

The assays were performed in PBS containing 0.1% BSA to reduce nonspecific binding. 2 mg of the various SYNSORBs were incubated for 30 min on an end-over-end rotator with approximately 20,000 dpm of the iodinated SLTI prepared in Preparation B above (specific activity, 2.2 x 10⁷ dpm/µg, CD₅₀ in the Verocytotoxicity assay, 0.4 pg/ml), in 0.5 ml PBS/BSA). The SYNSORBs were then washed with 3 x 1 ml portions of PBS BSA to remove unbound counts. The derivatized SYNSORBs were counted in an LKB Rackgamma model 1270 Gamma Counter.

The results are shown in Table 1.

**Table 1**

| SYNSORB | % SLTI Bound |
|---|---|
| Pk-tri | 93 |
| # 115 | 21 |
| Glc | 9 |
| ASA | 5 |

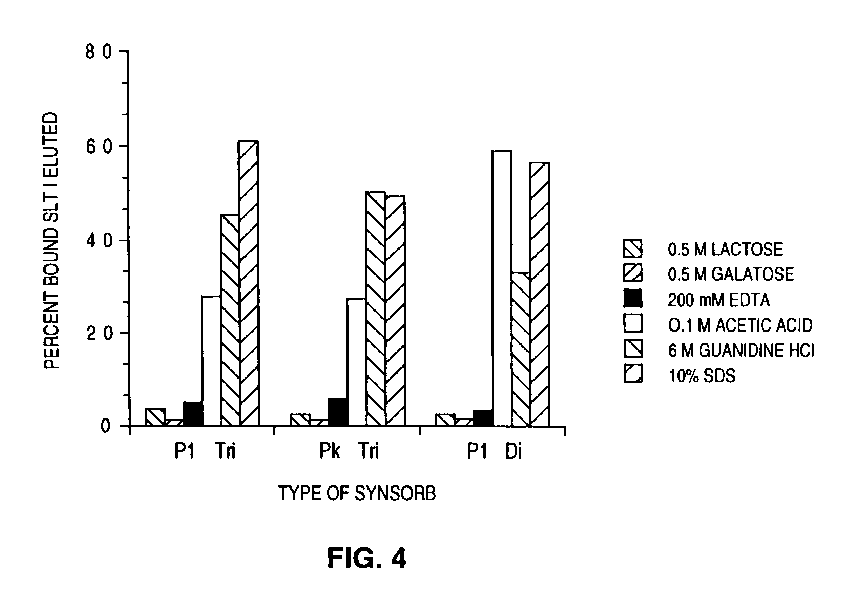

The SLT bound to Pk-tri SYNSORB could be partially released using 0.1 M acetic acid, 6 M guanidine HCl, or by heating in boiling water bath for 30 min in 10% SDS. However, neither 0.5 M lactose, 0.5 M galactose, or 0.2 M EDTA could displace the bound SLTI (Figure 4).

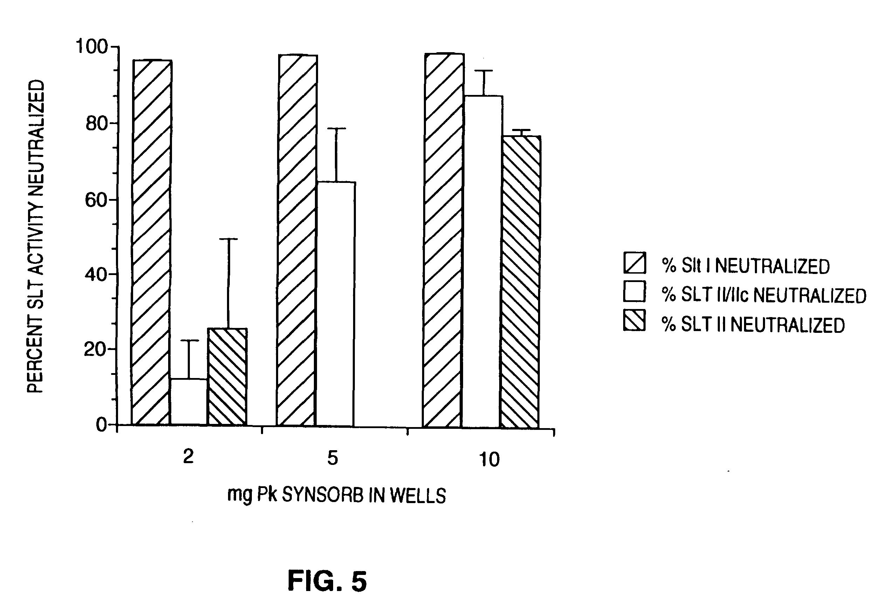

Subsequent experiments showed that 2 mg of Pk-tri neutralized approximately 90% of the activity in *E. coli* H19 (SLTI) but about 10 mg Pk-tri SYNSORB was required to neutralize the activity of the *E. coli* 32511 (SLTII/SLTIIc) or *E. coli* C600/933W (SLTII) to a similar extent (Figure 5).

### Example 3

### Performance Under Digestive Tract Conditions

Pk Trisaccharide SYNSORB was incubated for the various times at 37°C in 0.01 M HCl to simulate conditions in the stomach, then washed extensively in PBS to remove the HCl. The HCl-treated SYNSORB was then tested for SLTI and SLTII neutralizing activity in the Vero cytotoxicity assay as described hereinabove,and by Armstrong, G.D. *et al.,* J Infect Dis (1991) 164:1160-1167.

Briefly, *E. coli* 026:H11 (SLTI) or 0157:H⁻ (SLTII) were grown overnight at 37°C on Tryptic Soy Agar (TSA). The bacteria from 5 plates were harvested in 3.0 ml PBS containing 0.1 mg/ml Polymyxin B sulfate. The resulting suspension was then clarified by centrifugation.

Five mg of Pk trisaccharide SYNSORB was mixed with approximately 1 ml of polymyxin extract of the bacterial strains listed in the table below. Data represent the average of two independent determinations, each performed in duplicate. Values in brackets give the range for each value.

**Table 2**

| *Ability of HCl-Treated Pk Trisaccharide SYNSORB to Neutralize SLT Activity in the Vero Cell Assay* | | | |
|---|---|---|---|
| HC1 | Percent SLT Activity Neutralized | | |
| Incubation Time (Hours at 37°C) | 026:H11 (SLTI) | 0157:H7 (SLTII/IIc) | C600 (SLTII) |
| 0 | 94 (4) | 88 (5) | 55 (10) |
| 1 | 94 (6) | 80 (2) | 66 (5) |
| 4 | 96 (5) | 91 (8) | 73 (2) |
| 18 | 94 (6) | 93 (6) | 70 (7) |

As shown in Table 2, incubation with HCl does not appreciably diminish neutralizing activity. To simulate intestinal conditions, various SYNSORBs were incubated for 2 hours at 37°C in buffer or in rat intestinal sacs. The incubated SYNSORBs were assayed for neutralizing activity against SLTI and SLTII, generally as described above. Briefly, SYNSORBs recovered from the rat intestines were sonicated for 30 to 60 seconds in a Branson Model B-220 Ultrasonic Cleaner to disrupt clumps of aggregated material. The sonicated SYNSORBs were then washed 4 times with 5 ml of double distilled, deionized H₂O and dried under vacuum. Control SYNSORBs were treated in a similar manner. The polymyxin-extract described above was diluted to 8 ml with PBS. Five mg of SYNSORB was added to 0.9 ml portions of the diluted polymyxin extract. These were then incubated at room temperature for 1 hour on an end-over-end rotator. The resulting supernatant solutions were analyzed for SLTI or SLTII activity. Percent neutralization was calculated relative to the CD₅₀s of polymyxin extracts incubated with the ASA control SYNSORB. These results are shown in Tables 3-6.

Table 3 shows the results of neutralization of SLTI activity by SYNSORB incubated in buffer; Table 4 shows neutralization of SLTI activity by SYNSORB incubated in intestinal sacs; Table 5 shows neutralization of SLTII activity by SYNSORB incubated in buffer; and Table 6 shows neutralization of SLTII activity by SYNSORB incubated in rat intestinal sacs.

**Table 3**

| SYNSORB | Percent SLTI Activity Neutralized^{a} |
|---|---|
| ASA SYNSORB^{b} | 0 |
| Pₗ Disaccharide | 93 (88 - 97) |
| Pₗ Trisaccharide | 98 (96 - 100) |

| | |
|---|---|
| a. Average of duplicate determinations. Range in brackets. | |
| b. Control SYNSORB containing only the acetylated silylaminated (ASA) hydrophobic 8-methoxycarbonyloctyl linkage arm. | |

**Table 4**

| SYNSORB | Percent SLTI Activity Neutralized^{a} |
|---|---|
| ASA SYNSORB | 0 |
| Pₗ Disaccharide | 82 ± 6 |
| Pₗ Trisaccharide | 98 ± 2 |

| | |
|---|---|
| a. Average of triplicate determinations ± standard deviation of the mean. | |

**Table 5**

| n SYNSORB | Percent SLTII Activity Neutralized^{a} |
|---|---|
| ASA SYNSORB | 0 |
| Pₗ Disaccharide | 84 |
| Pₗ Trisaccharide | 98 |

| | |
|---|---|
| a. Results of one determination. | |

**Table 6**

| SYNSORB | Percent SLTII Activity Neutralized^{a} |
|---|---|
| ASA SYNSORB | 0 |
| Pₗ Disaccharide | 65 ± 9^{a} |
| Pₗ Trisaccharide | 96^{b} |

| | |
|---|---|
| a. Average of triplicate determinations ± standard deviation of the mean. | |
| b. Results of one determination. | |

As shown above, neither the conditions of the stomach nor those of the small intestine are detrimental to the activity of the derivatized SYNSORBs in neutralizing SLTI or SLTII.

## Claims

1. A pharmaceutical composition useful in treating enteric infections mediated by shiga like toxin (SLT) which composition comprises as active ingredient a pharmaceutically acceptable solid inert affinity support capable of being eliminated from the gastrointestinal tract which support has an affinity ligand covalently attached thereto through a spacer arm wherein said ligand is characterized as an oligosaccharide containing, at the non-reducing terminus thereof, the αGal(1-4)βGal(1-4)βGlcNAc trisaccharide or the αGal(1-4)βGal(1-4)βGlc trisaccharide which binds the SLT, in admixture with a pharmaceutically acceptable excipient.

2. The pharmaceutical composition of claim 1, wherein said oligosaccharide is the αGal(1-4)βGal(1-4)βGlcNAc trisaccharide or the αGal(1-4)βGal(1-4)βGlc trisaccharide.

3. The pharmaceutical composition of claim 1 or 2, wherein said spacer arm corresponds to -(CH₂)₈C(O)-.

4. A pharmaceutical composition according to claim 1, 2 or 3, wherein the spacer arm is of a corresponding length to -(CH₂)₈C(O)- and has a functional group at one terminus which reacts with the oligosaccharide affinity ligand and a functional group at the other terminus which reacts with the solid support.

5. An agent for use in a method of treating a subject to prevent or ameliorate an enteric infection mediated by SLT, wherein said agent is a pharmaceutically acceptable solid inert affinity support capable of being eliminated from the gastrointestinal tract which support has an affinity ligand covalently attached thereto through a spacer arm wherein said ligand is characterized as an oligosaccharide containing, at the non-reducing terminus thereof, the αGal(1-4)βGal(1-4)βGlcNAc trisaccharide or the αGal(1-4)βGal(1-4)βGlc trisaccharide which binds the SLT,

6. An agent for use as claimed in claim 5, wherein said oligosaccharide is the αGal(1-4)βGal(1-4)βGlcNAc trisaccharide or the αGal(1-4)βGal(1-4)βGlc trisaccharide.

7. An agent for use as claimed in claim 5 or 6, wherein said spacer arm corresponds to -(CH₂)₈C(O)-.

8. An agent for use as claimed in claim 5, 6 or 7, wherein the spacer arm is of a corresponding length to -(CH₂)₈C(O)- and has a functional group at one terminus which reacts with the oligosaccharide affinity ligand and a functional group at the other terminus which reacts with the solid support.

## Patentansprüche

1. Pharmazeutische Zusammensetzung zur Behandlung enterischer Infektionen, welche vom Shiga-like Toxin (SLT) herkommen, enthaltend als aktiven Wirkstoff einen festen, inerten, pharmazeutisch akzeptablen Affinitätsträger, der vom Magen-Darmtrakt ausgeschieden werden kann, wobei am Träger kovalent über eine Abstandsgruppe ein Affinitätsligand gebunden ist, welcher gekennzeichnet ist durch ein Oligosaccharid, das am nicht-reduzierenden Ende das Trisaccharid αGal(1-4)-βGal(1-4)βGlcNac oder das Trisaccharid αGal(1-4)βGal(1-4)βGlc besitzt, welches an SLT bindet, in einem Gemisch mit einem pharmazeutischen akzeptablen Exzipienten.

2. Pharmazeutische Zusammensetzung nach Anspruch 1, wobei das Oligosaccharid das Trisaccharid αGal(1-4)-βGal(1-4)βGlcNAc oder das Trisaccharid αGal(1-4)-βGal(1-4)βGlc ist.

3. Pharmazeutische Zusammensetzung nach Anspruch 1 oder 2, wobei die Abstandsgruppe -(CH₂)₈C(O) - entspricht.

4. Pharmazeutische Zusammensetzung nach Anspruch 1, 2 oder 3, wobei die Länge der Abstandsgruppe - (CH₂)₈C(O) - entspricht und die Abstandsgruppe an einem Ende eine funktionelle Gruppe trägt, die mit dem Oligosaccharid-Affinitätsliganden umgesetzt werden kann, und am anderen Ende eine funktionelle Gruppe trägt, die mit dem festen Träger umgesetzt werden kann.

5. Mittel zur Verwendung in einem Verfahren zur Behandlung von Jemandem zur Vermeidung und Linderung einer enterischen Infektion, die von SLT herkommt, wobei das Mittel ein fester, inerter, pharmazeutisch akzeptabler Affinitätsträger ist, der vom Magen-Darmtrakt ausgeschieden werden kann und an dem über eine Abstandsgruppe kovalent ein Affinitätsligand gebunden ist, welcher gekennzeichnet ist durch ein Oligosaccharid, das am nicht-reduzierenden Ende das Trisaccharid αGal(1-4)βGal(1-4)βGlcNAc oder das Trisaccharid αGal(1-4)βGal(1-4) βGlc besitzt, welches an SLT bindet.

6. Mittel zur Verwendung nach Anspruch 5, wobei das Oligosaccharid das Trisaccharid αGal(1-4)βGal(1-4)βGlcNAc oder das Trisaccharid αGal(1-4)βGal(1-4)βGlc ist.

7. Mittel zur Verwendung nach Anspruch 5 oder 6, wobei die Abstandsgruppe (CH₂)₈C(O) - entspricht.

8. Mittel zur Verwendung nach Anspruch 5, 6 oder 7, wobei die Abstandsgruppe eine Länge wie -(CH₂)₈C(O)- besitzt und an einem Ende eine funktionelle Gruppe trägt, die mit dem Oligosaccharid-Affinitätsliganden reagieren kann sowie am anderen Ende eine funktionelle Gruppe, die mit dem festen Träger reagieren kann.

## Revendications

1. Composition pharmaceutique utile dans le traitement d'infections entériques à médiation par la toxine de type Shiga (SLT), composition qui comprend comme ingrédient actif un support d'affinité inerte solide pharmaceutiquement acceptable pouvant être éliminé du tractus gastro-intestinal, support qui comprend un ligand d'affinité fixé par covalence à celui-ci par une branche intercalaire, dans laquelle ledit ligand est caractérisé en tant qu'oligosaccharide contenant, à son extrémité non réductrice, le trisaccharide αGal(1-4)βGaI(1-4)BGlcNAc ou le trisaccharide αGal(1-4)βGal(1-4)BGlc qui se lie à la SLT, en mélange avec un excipient pharmaceutiquement acceptable.

2. Composition pharmaceutique suivant la revendication 1, dans laquelle l'oligosaccharide est le trisaccharide αGal(1-4)βGal(1-4)BGlcNAc ou le trisaccharide αGal(1-4)βGal(1-4)BGlc.

3. Composition pharmaceutique suivant la revendication 1 ou 2, dans laquelle la branche intercalaire correspond à -(CH₂)₈C(O)-.

4. Composition pharmaceutique suivant la revendication 1, 2 ou 3, dans laquelle la branche intercalaire est d'une longueur correspondante à - (CH₂)₈C(O)- et comporte un groupe fonctionnel à une extrémité qui réagit avec le ligand oligosaccharide d'affinité et un groupe fonctionnel à l'autre extrémité qui réagit avec le support solide.

5. Agent destiné à être utilisé dans une méthode de traitement d'un sujet pour la prévention ou l'atténuation d'une affection entérique à médiation par la SLT, ledit agent étant un support d'affinité inerte solide pharmaceutiquement acceptable pouvant être éliminé du tractus gastro-intestinal, support qui comporte un ligand d'affinité fixé par covalence à celui-ci par une branche intercalaire, dans lequel ledit ligand est caractérisé en tant qu'oligosaccharide contenant, à son extrémité non réductrice, le trisaccharide αGal(1-4)βGal(1-4)BGlcNAc ou le trisaccharide αGal(1-4)βGal(1-4)BGlc qui se lie à la SLT.

6. Agent destiné à être utilisé suivant la revendication 5, dans lequel l'oligosaccharide est le trisaccharide αGal(1-4)βGal(1-4)BGlcNAc ou le trisaccharide αGal(1-4)βGal(1-4)BGlc.

7. Agent destiné à être utilisé suivant la revendication 5 ou 6, dans lequel la branche intercalaire correspond à -(CH₂)₈C(O)-,

8. Agent destiné à être utilisé suivant la revendication 5, 6 ou 7, dans lequel la branche intercalaire est d'une longueur correspondante à -(CH₂)₈C(O)- et possède un groupe fonctionnel à une extrémité qui réagit avec le ligand oligosaccharide d'affinité et un groupe fonctionnel à l'autre extrémité qui réagit avec le support solide.
